# EUROPÄISCHE PATENTSCHRIFT

(11) **EP 4 041 384 B1**
(45) Veröffentlichungstag und Bekanntmachung des Hinweises auf die Patenterteilung: **20.08.2025**
(21) Anmeldenummer: 21770149.9
(22) Anmeldetag: 26.08.2021
(51) Int. Cl.: A61N 5/06, A61H 19/00

(54) **LIEBESKUGEL**
LOVE BALL
BOULE D'AMOUR

(30) Priorität: 20.11.2020 DE 202020106672 U
(43) Veröffentlichungstag der Anmeldung: 17.08.2022
(73) Patentinhaber: MEDLight GmbH, 32051 Herford (DE)
(72) Erfinder: WÄCHTER, Herta, 32105 Bad Salzuflen (DE); STOLZE, Jürgen, 41541 Dormagen (DE)
(74) Vertreter: Schleitzer, Dirk-Karsten
(86) Internationale Anmeldenummer: PCT/EP2021/073571
(87) Internationale Veröffentlichungsnummer: WO 2022/106082

(56) Entgegenhaltungen:
- EP-A1- 2 316 405
- WO-A1-2014/166993
- WO-A1-2017/058957
- DE-A1- 102016 211 295

## Beschreibung

Die Erfindung betrifft eine Liebeskugel mit einem Behandlungskörper zum vorzugsweise vollständigen Einführen in ein Vaginal- oder Rektallumen.

Bei sogenannten "Liebeskugeln", die auch unter der Bezeichnung "Kegel Ball" bekannt sind, handelt es sich um Massagevorrichtungen, die nicht nur als Erotikspielzeug verwendet werden, sondern auch als intravaginale Behandlungsvorrichtung bei der therapeutischen Behandlung der Beckenbodenmuskulatur, wie beispielsweise in der EP 0 955 024 A2 beschrieben. Solche Massagevorrichtungen werden bei bestimmungsgemäßer Verwendung wenigstens abschnittsweise in einen vaginalen Hohlraum eingeführt und bis zum Erreichen einer Massagewirkung temporär in diesem belassen, insbesondere, um die Beckenbodenmuskulatur zu massagieren bzw. zu trainieren. Vaginalkonen, so der medizinische Begriff für Liebeskugeln, stärken die Muskeln des Beckenbodens und erhöhen dadurch nicht nur die sexuelle Erlebnisfähigkeit, sondern können auch Inkontinenz bekämpfen. Das Training der Beckenbodenmuskulatur zur Therapie oder Prophylaxe von Belastungsinkontinenz erfolgt durch unwillkürliche Kontraktionen, um das Gewicht des Vaginalkonus intravaginal zu halten. Andere Bestrahlungsvorrichtung für vaginale Behandlungen ist aus WO 2014/166993 A1 bekannt.

Aufgabe der vorliegenden Erfindung ist es, eine Liebeskugel der zuvor beschriebenen Art bereitzustellen, die über die Verwendung als Erotikspielzeug und insbesondere als Massagevorrichtung zur therapeutischen Behandlung der Beckenbodenmuskulatur hinaus eine weitergehende therapeutische Funktionalität aufweist, wobei die Liebeskugel als kompaktes Gerät für den Verbraucher und für die Behandlung bzw. Therapie in häuslicher Umgebung vorgesehen ist. Bei der bestimmungsgemä-ßen Verwendung, wenn der Behandlungskörper der Liebeskugel in einen vaginalen oder rektalen Hohlraum eingeführt ist, wird dieser temporär bis zum Erreichen einer therapeutischen Wirkung zumindest teilweise, vorzugsweise vollständig, in dem Hohlraum belassen, wobei sich die Liebeskugel durch einen angenehmen Tragezustand auszeichnen und auch geeignet sein soll, beim Träger angenehme Reaktionen bis hin zur sexuellen Stimulation auszulösen.

Die vorgenannte Aufgabe wird durch eine Liebeskugel mit den Merkmalen von Anspruch 1 gelöst. Vorteilhafte Ausgestaltungen der Erfindung sind Gegenstand der abhängigen Ansprüche.

Erfindungsgemäß ist vorgesehen, dass die Liebeskugel eine in den Behandlungskörper integrierte Bestrahlungseinrichtung, insbesondere eine LED-Bestrahlungseinrichtung, und eine insbesondere in den Behandlungskörper integrierte Stromquelle als Energiespeicher zur Stromversorgung der Bestrahlungseinrichtung aufweist, wobei die Liebeskugel betriebsbereit ist, während sie sich in der Körperöffnung befindet. Zudem weist die Liebeskugel eine insbesondere ebenfalls in den Behandlungskörper integrierte Steuerschaltung zur Steuerung oder Regelung der Bestrahlungseinrichtung auf, wobei die Bestrahlungseinrichtung konfiguriert ist zur Emission von Licht mit einer Wellenlänge zwischen 600 nm und 750 nm und zur Bestrahlung mit einer maximalen Bestrahlungsstärke zwischen 0,1 mW/cm² 1,5 mW/cm².

Der Erfindung liegt der Grundgedanke zugrunde, eine Liebeskugel zur Fotobiostimulation umliegenden Gewebes einzusetzen, wobei die Lichtemission eines oder mehrerer Lichtemitter der Liebeskugel erfindungsgemäß in einem Bereich liegen soll, der geeignet ist, Apoptose und Zelltod zu verhindern, Proliferation, Migration und Kollagensynthese von Fibroblasten zu stimulieren, Entzündungs- und Antioxidationsreaktionen zu modulieren und/oder Angiogenese oder Gewebereparatur insbesondere im Vaginalgewebe zu begünstigen. Die erfindungsgemäße Liebeskugel kombiniert somit die bei der Anwendung der Liebeskugel erreichte mechanische Anregung und Stärkung der Beckenbodenmuskulatur mit einer Gewebeverjüngung durch Fotobiostimulation.

Ein wesentlicher Vorteil der vorliegenden Erfindung gegenüber bekannten medizinischen Behandlungsgeräten zur Fotobiostimulation liegt darin begründet, dass die Beleuchtung erfindungsgemäß mit sehr geringen Bestrahlungsstärken bzw. Fluenzraten erfolgt, wobei die Bestrahlungsstärke in der Einheit [W/cm²] angegeben wird und die durch Strahlung übertragene Energie in [J] bezogen auf die Empfängeroberfläche des den Behandlungskörper umgebenden Gewebes und die Zeit beschreibt. Erfindungsgemäß erfolgt eine Beleuchtung mit niedrigen Bestrahlungsstärken, so dass die Beleuchtung über einen relativ langen Zeitraum erfolgt, um eine gewünschte Lichtdosis zu erreichen, die zur Erzielung einer therapeutischen Wirkung erforderlich ist. Durch die Beleuchtung mit niedrigen Bestrahlungsstärken wird verhindert, dass bei der Anwendung der Liebeskugel Schmerzen auftreten, woraus in Verbindung mit einer geeigneten Formgebung und einem geeigneten Gewicht der Liebeskugel ein angenehmer Tragezustand resultieren kann. Dadurch wird es hochwahrscheinlich, dass regelmäßige Behandlungen stattfinden.

Nach der Geburt eines Kindes oder mit zunehmenden Alter können Frauen eine Schwächung oder Entspannung ihrer vaginalen Muskulatur erfahren. Diese Entspannung des Vaginalmuskels ist als vaginal relaxation syndrom (VRS) oder Vaginalwanddehnung bekannt und wirkt sich negativ auf den Geschlechtsverkehr aus, kann Intimitäts- und Selbstwertprobleme verursachen und zu Harninkontinenz führen. Herkömmliche Lösungen zur Straffung des entspannten Vaginalmuskels umfassen Kegelübungen und Vaginalcremes, die jedoch im Allgemeinen unwirksam sind. Kostspielige und invasive Verfahren zur Vaginalverjüngung sind eine weitere Option, bieten jedoch auch keine sichere, komfortable und erschwingliche Option für die Vaginalverjüngung. Klinische Behandlungsgeräte zum Einführen in die Vagina sind ebenfalls erhältlich. Die Fähigkeit dieser herkömmlichen Geräte, VRS tatsächlich zu behandeln, scheint jedoch begrenzt zu sein. Darüber hinaus sind diese Geräte für die Behandlung in einer klinischen Umgebung vorgesehen und weder als kompaktes Gerät für den Verbraucher- oder Heimgebrauch konzipiert, noch als angenehm für die Frau zu verwenden, wodurch es weniger wahrscheinlich ist, dass regelmäßige Behandlungen stattfinden, und die Wirksamkeit der Geräte abnimmt.

Mit der Erfindung wird insbesondere eine Liebeskugel zur Anwendung als Therapiervorrichtung für eine kombinierte Verjüngungs- und mechanische Beckenbodenmuskulatur-Therapie vorgeschlagen, wobei eine kontrollierte Strahlungsenergie auf das umgebende, insbesondere die Vaginalschleimhaut umgebende, subdermale Bindegewebe abgegeben wird, was zu einer kollagenen Umgestaltung des umgebenden Bindegewebes in einem Prozess führt, der mit dem Schmelzen des Kollagens beginnt und mit Reaktionen endet, die eine signifikante Straffung des Lumens bewirken.

Durch die Formgebung und das Gewicht des Behandlungskörpers der Liebeskugel werden während der Therapie angenehme Gefühle ausgelöst, wobei die Liebeskugel einfach zu bedienen ist und eine längere Nutzung der Liebeskugel gefördert wird. Der Behandlungskörper der Liebeskugel kann derart ausgestaltet sein und eine Formgebung derart aufweisen, dass lediglich lokalisierte Bereiche intravaginalen Gewebes und nicht die gesamte Länge des Vaginallumens behandelt werden. Vorzugsweise ist jedoch der Behandlungskörper derart dimensioniert und weist eine Formgebung derart auf, dass er im Wesentlichen vollständig in das Vaginallumen einführbar ist. Die erfindungsgemäße Liebeskugel kann dabei zusammen mit Schmiermitteln, die an sich aus dem Stand der Technik bekannt sind, verwendet werden.

Das Bestrahlen des umgebenden Gewebes mit Licht im erfindungsgemäß vorgesehenen Wellenlängenbereich führt zu einem begrenzten Eindringen von Licht in das Gewebe, so dass eine Behandlung oberflächiger Gewebeveränderungen möglich ist, ohne empfindliche Strukturen unterhalb des oberflächennahen Gewebes zu beschädigen. Es ist davon auszugehen, dass die Verwendung der erfindungsgemäßen Liebeskugel auch zur Behandlung gynäkologischer Probleme eingesetzt werden kann, insbesondere zur Behandlung der zervikalen Dysplasie. Dysplasie ist ein abnormales Wachstum von Zellen. Dysplasie von Gebärmutterhals ist ein Begriff, der verwendet wird, um das Auftreten abnormaler Zellen auf der Oberfläche des Gebärmutterhalses zu beschreiben. Während Dysplasie selbst keine gesundheitlichen Probleme verursacht, wird sie als Präkanzerose angesehen. Wenn Dysplasie unbehandelt bleibt, kann sie sich zu einer frühen Form von Krebs entwickeln, die schließlich zu Gebärmutterhalskrebs führen kann.

Schließlich kann die mit der erfindungsgemäßen Liebeskugel erreichte Bestrahlung insbesondere von vaginalem Körpergewebe auch Mikroorganismen, insbesondere Bakterien und Pilze, abtöten oder deren Wachstum beeinträchtig, so dass die erfindungsgemäße Liebeskugel auch im Rahmen einer Behandlung von insbesondere Pilz- oder bakteriellen Infektionen eingesetzt werden kann.

Mit der Erfindung wird der Anwendungsbereich von Liebeskugeln über die bekannte Verwendung zur therapeutischen Behandlung der Beckenbodenmuskulatur, die auf das Gewicht und die Form der Liebeskugel sowie deren Dimensionierung zurückzuführen ist, hinaus erweitert auf neue therapeutische Anwendungsbereiche. So lässt sich die erfindungsgemäße Liebeskugel insbesondere zur Gewebeverjüngung durch fotodynamische Therapie in Körperhohlräumen, mit oder ohne Verwendung von Photosensibilisatoren, einsetzen, insbesondere zur vaginalen Verjüngung durch Anregung der Kollagenneubildung und/oder der Gewebeerneuerung, und/oder zur Behandlung von Gewebeveränderungen in Körperhohlräumen, insbesondere zur Behandlung der zervikalen Dysplasie, und/oder zur Behandlung von insbesondere bakteriellen oder Pilzinfektionen.

Als Lichtemitter bzw. Leuchtmittel für die Bestrahlung ist vorzugsweise wenigstens eine lichtemittierende Diode (LED) vorgesehen, was die Bestrahlung mit einer definierten Wellenlänge ermöglicht und die Verwendung von beispielsweise optischen Filtern, um bestimmte ungewünschte Wellenlängen herauszufiltern und eine Bestrahlung im angestrebten Wellellängenbereich sicherzustellen, entbehrlich macht.

Die Bestrahlungsstärke ist erfindungsgemäß bezogen auf einen Abstand des Behandlungskörpers der Liebeskugel von dem umgebenden Empfängergewebe von weniger als 5 mm, vorzugsweise von weniger als 1 mm. Insbesondere ist die Bestrahlungsstärke bezogen auf einen Zustand, in dem der Bestrahlungskörper mit seiner Außenfläche vollumfänglich und/oder vollflächig gegen das umgebende Gewebe anliegt.

Im Übrigen kann die erfindungsgemäße Liebeskugel eine Datenkommunikationseinrichtung aufweisen, die zum Senden und/oder Empfangen von Funksignalen ausgebildet ist und einen Datentransfer mit einer entfernten Bedieneinrichtung zur Steuerung der Bestrahlungseinrichtung ermöglicht. Die Datenkommunikationseinrichtung kann ein Antennenelement zum Senden und Empfangen von Funksignalen umfassen. Das Antennenelement kann für das Senden und Empfangen von Funksignalen nach Bluetooth, NFC (Near Field Communication), WLAN, GSM, UMTS, LTE oder einem anderen mobilen Datenübertragungsstandard geeignet sein.

Vorzugsweise ist eine Bestrahlung mit Licht einer Wellenlänge zwischen 620 und 640 nm, vorzugsweise von 630 nm, vorgesehen, bezogen auf eine zulässige Peakabweichung von ± 3 nm. In Zusammenhang mit der Erfindung durchgeführte Versuche an Probanden haben gezeigt, dass sich in diesem Wellenlängenspektrum eine sehr erfolgversprechende Fotobiostimulation des Gewebes erreichen lässt.

Die Bestrahlungseinrichtung ist darüber hinaus vorzugsweise zur Beleuchtung mit einer Bestrahlungsstärke zwischen 0,1 mW/cm², vorzugsweise zwischen 0,25 und 1,0 mW/cm², konfiguriert, bezogen auf die Empfängerfläche des den Behandlungskörper der Liebeskugel im eingeführten Zustand umgebenen Gewebes. Durch die erfindungsgemäß vorgesehene niedrige Bestrahlungsstärke ist eine unerwünschte Schädigung des umliegenden Gewebes bei der Behandlung mit der erfindungsgemäßen Liebeskugel ausgeschlossen und eine schmerzfreie Therapie sichergestellt.

Um Gewebeschäden jedenfalls sicher ausschließen zu können, ist die Steuerschaltung bei der Ausführungsform der Erfindung zum automatischen Abschalten der Bestrahlung bei Erreichen einer Bestrahlungsenergie von weniger als 5,0 J, weiter vorzugsweise von weniger als 2,0 J, besonders bevorzugt von 0,5 bis 1,5 J, und/oder nach einer Bestrahlungsdauer von höchstens 60 min, vorzugsweise von höchstens 30 min, weiter vorzugsweise von höchstens 20 Minuten, ausgebildet. Die Bestrahlungsdauer kann hierbei von einer Person frei oder in diskreten Schritten innerhalb vorgegebener Werte einstellbar sein oder es kann wenigstens eine feste Beleuchtungsdauer unveränderbar vorgegeben und in der Steuerschaltung hinterlegt sein.

Um eine hohe therapeutische Wirkung bei Anwendung der erfindungsgemäßen Liebeskugel zur Behandlung der Beckenbodenmuskulatur sicherzustellen, sieht die Erfindung vorzugsweise vor, dass der Behandlungskörper im Benutzungszustand ein Gesamtgewicht von wenigstens 30 g, vorzugsweise von wenigstens 40 g, weiter vorzugsweise von 50 g, besonders bevorzugt von 60 g, und/oder von weniger als 100 g aufweist. Das Gesamtgewicht bezieht sich insbesondere auf einen Zustand mit eingesetztem Stromspeicher bzw. eingesetzter Batterie.

Es kann zweckmäßig sein, in dem Behandlungskörper einen Aufnahmeraum zur Aufnahme wenigstens einer austauschbaren Beschwerungsmasse zu integrieren, vorzugsweise zur Aufnahmen unterschiedlich schwerer Beschwerungsmassen. Durch Einsatz unterschiedlich schwerer Beschwerungsmassen und deren bedarfsweise Integration in den Behandlungskörper lässt sich die Muskulaturbeanspruchung beim Tragen der erfindungsgemäßen Liebeskugel bedarfsweise verändern, um die therapeutische Wirkung zu erhöhen und/oder einen angenehmen Tragezustand zu erreichen.

Der Behandlungskörper kann wenigstens eine austauschbare Beschwerungsmasse vorsehen, wobei, vorzugsweise, die Beschwerungsmasse ein Gewicht zwischen 5 g und 15 g, vorzugsweise 10 g, aufweisen kann. Das Bauteilgewicht des Behandlungskörpers ohne Beschwerungsmasse kann vorzugsweise zwischen 30 g und 60 g, weiter vorzugsweise 40 g, betragen.

Bei der Beschwerungsmasse kann es sich um eine Batterie bzw. einen elektrischen Stromspeicher handeln oder die Beschwerungsmasse kann auch nicht stromspeichernd sein. Es können Batterien mit unterschiedlichem Gewicht eingesetzt werden und/oder zusätzlich zur Batterie weitere Beschwerungsmassen.

Um eine zu starke Erwärmung der Außenflächen des Behandlungskörpers infolge des Betriebs der Bestrahlungseinrichtung ausschließen zu können, kann eine Temperaturmesseinrichtung (Sensor) zur Erfassung der Oberflächentemperatur der Außenfläche des Behandlungskörpers vorgesehen sein, wobei, vorzugsweise, die Steuerschaltung zum automatischen Abschalten der Bestrahlung bei Erreichen einer Oberflächentemperatur von höchstens 45 °C, vorzugsweise von höchstens 42 °C, weiter vorzugsweise von höchstens 40 °C oder weniger, ausgebildet sein kann. Damit lassen sich Gewebeschäden und Schmerzen bei der Anwendung der Liebeskugel wirkungsvoll verhindern.

Durch entsprechende Dimensionierung des Bestrahlungskörpers, d.h. das Verhältnis von Oberfläche zu Volumen, in Abhängigkeit von der Bestrahlungsstärke, die Wärmespeicherkapazität des Bestrahlungskörpers einschließlich aller integrierten Bauteile und durch die vorgesehene Zeitdauer der Bestrahlung lässt sich die Oberflächentemperatur des Bestrahlungskörpers begrenzen, die beim Betrieb der Bestrahlungseinrichtung maximal erreicht werden kann. Es ist zweckmäßig, wenn die Oberflächentemperatur des Behandlungskörpers bei unterbrechungsloser Bestrahlung des umgebenden Gewebes mit einer maximalen Bestrahlungsstärke über einen Zeitraum von weniger als 60 min, vorzugsweise von weniger als 30 Minuten, weiter vorzugsweise von weniger als 20 Minuten, einen Maximalwert von 45 °C, vorzugsweise von 42 °C, weiter vorzugsweise von 40 °C, nicht überschreitet. Dies erfordert eine Abstimmung des Oberflächen- zu Volumenverhältnisses, der Wärmespeicherkapazität des Behandlungskörpers und der integrierten Bauteile sowie der vorgesehenen Zeitdauer der Bestrahlung.

Die Lichtemission kann kontinuierlich erfolgen oder die Bestrahlungseinrichtung wird gepulst betrieben. Beim gepulsten Betrieb wird das Licht in zeitlich begrenzten Portionen (Pulsen) emitiert, wobei die Pulslänge so gewählt sein kann, dass der Behandlungskörper an seiner Oberfläche einen Maximalwert von 45 °C, vorzugsweise von 42 °C, weiter vorzugsweise von 40 °C, nicht überschreitet.

Im Übrigen kann die Steuerschaltung konfiguriert sein zum automatischen Abschalten der Bestrahlung nach Erreichen einer Bestrahlungsdauer von weniger als 60 min, vorzugsweise von weniger als 30 min, weiter vorzugsweise von weniger als 20 min, und/oder bei Erreichen einer emittierten Bestrahlungsenergie von weniger als 37 J/cm², vorzugsweise von weniger als 5,0 J/cm², weiter vorzugsweise von weniger als 2,0 J/cm², besonders bevorzugt von 0,5 J/cm² bis 1,5 J/cm².

Um eine zu starke Temperaturerhöhung des Behandlungskörpers aufgrund der Lichtemission zu verhindern, kann eine Kühleinrichtung zur Kühlung des Behandlungskörpers vorgesehen sein. Die Kühlung kann durch erzwungene Konvektion erfolgen, wobei Kühlluft von außerhalb der Körperöffnung zum Behandlungskörper geleitet und erwärmte Kühlluft aus dem Behandlungskörper in die Umgebung zurückgeführt wird. Der Behandlungskörper kann auch eine Kavität bzw. einen innenliegenden Hohlraum zur Aufnahme eines Kühlmediums, wie beispielsweise Eis, aufweisen.

Die Beleuchtungseinrichtung weist vorzugsweise mehrere LEDs auf, wobei, vorzugsweise, eine Anordnung der LEDs und/oder ein Abstrahlwinkel der LEDs derart vorgesehen sein kann, dass eine vollumfängliche Lichtabstrahlung insbesondere um eine Längsachse des Behandlungskörpers und/oder zu den Seiten hin, bezogen auf einen eingeführten Zustand des Behandlungskörpers, erfolgt.

Beispielsweise können mehrere LEDs der Anzahl n jeweils mit einem Abstrahlwinkel von 360°/n vorgesehen sein, beispielsweise drei LEDs jeweils mit einem Abstrahlwinkel von 120°. Durch eine Mehrzahl von LEDs lässt sich die Einzelleistung jeder LED reduzieren, die für eine Fotobiostimulation erforderlich ist. Die LEDs können auf einem ringförmigen Träger angeordnet sein, der koaxial zu einer Längsachse des Behandlungskörpers angeordnet sein kann. Der Begriff "Abstrahlwinkel" beschreibt die winkelabhängige Lichtstärke. Im Falle gerichteter Lichtquellen meint der Abstrahlwinkel jenen Winkel, der von den seitlichen Punkten, mit der das Licht vom Leuchtmittel abstrahlt, noch mit halber Maximal-Lichtstärke eingeschlossen wird. Der Abstrahlwinkel gibt also an, in welchem Winkel das Licht nach vorne abgestrahlt wird. Die volle Lichtstärke befindet sich dabei im Zentrum des von der LED erzeugten Lichtkreises, wobei bei einer bevorzugten Ausführungsform der Erfindung die maximale Lichtstärke in einer Richtung senkrecht zur Umfangsfläche des Behandlungskörpers abgestrahlt wird.

Die LEDs sind bei einer bevorzugten Ausführungsform der Erfindung in einer Ebene senkrecht zur Längsachse des Behandlungskörpers um die Längsachse des Behandlungskörpers in Umfangsrichtung gleichverteilt angeordnet, wobei die maximale Lichtstärke radial nach außen abgestrahlt wird.

Zur Stromversorgung kann wenigstens ein vorzugsweise austauschbarer und/oder wiederaufladbarer Speicher für elektrische Energie vorgesehen sein, insbesondere ein Speicher für elektrische Energie auf elektrochemischer Basis, weiter insbesondere eine herkömmliche Batterie, und/oder es kann eine Einrichtung zum kabellosen induktiven Wiederaufladen des Speichers vorgesehen sein.

Der Behandlungskörper weist vorzugsweise die Form eines Rotationskörpers auf, der ein einfaches Einführen des Behandlungskörpers in eine insbesondere vaginale Körperöffnung und einen sicheren Sitz des Behandlungskörpers mit den umgebenden Wänden der Körperöffnung gewährleistet. Vorzugsweise verjüngt sich der Rotationskörper in distaler Richtung hin. Der Rotationskörper kann konusförmig, birnenförmig, bananenförmig, phallus- oder kegel- oder ballförmig oder zylinderförmig mit halbkugelförmigen und/oder abgerundeten Stirnflächen sein. Der Rotationskörper kann einen kreisförmigen oder elliptischen Querschnitt aufweisen. Insbesondere ist der Rotationskörper länglich ausgebildet und erstreckt sich von einem proximalen, körpernahen Ende zu einem distalen, körperfernen Ende. Der Behandlungskörper kann an seinem proximalen Ende einen halbkugelförmigen Abschnitt mit größerem Durchmesser aufweisen, der in einen taillierten mittleren Abschnitt übergeht. Der taillierte mittlere Abschnitt kann in einen kugelförmigen Abschnitt mit kleinerem Durchmesser am distalen Ende des Behandlungskörpers übergehen, insbesondere stufenlos, so dass sich eine Birnenform ergibt. Weiter vorzugsweise wird der halbkugelförmige Abschnitt mit größerem Durchmesser am proximalen Ende des Behandlungskörpers und der taillierte Abschnitt gebildet durch ein proximales Gehäuseteil des Behandlungskörpers und der kugelförmige Abschnitt mit kleinerem Durchmesser am distalen Ende des Behandlungskörpers durch ein distales Gehäuseteil des Behandlungskörpers, wobei beide Gehäuseteile lösbar miteinander verbunden sein können.

Um die Reinigung zu erleichtern und eine Besiedlung mit Mikroorganismen bzw. deren Aufwachsen auf äußeren Oberflächen des Behandlungskörpers zu erschweren, weist der Behandlungskörper weiter vorzugsweise eine im Wesentlichen geschlossene, integrale Oberfläche auf. Der Behandlungskörper kann eine kontinuierliche (sprungfreie) Oberfläche aufweisen, die im wesentlichen vollflächig nach innen oder nach außen gerundet ist, sowohl mantelseitig als auch stirnseitig. Nach außen ist die Oberfläche des Behandlungskörpers vorzugsweise vollflächig geschlossen bzw. flüssigkeitsdicht, um das Eindringen von Körperflüssigkeit in den Behandlungskörper bei der Behandlung zu verhindern. Um eine Besiedlung mit Mikroorganismen zu erschweren, sind vorzugsweise keine Hinterschneidungen vorgesehen. Stattdessen weist der Behandlungskörper vorzugsweise lediglich gerundete Kanten auf oder ist kantenfrei gebildet.

An seinem distalen Ende kann der Behandlungskörper einen Rückholabschnitt zum Herausziehen des Behandlungskörpers aus dem Körper aufweisen. Der Rückholabschnitt kann langgestreckt, stabartig, biegbar und/oder elastisch ausgebildet sein und eine geringe Biegesteifigkeit aufweisen. Der Rückholabschnitt kann grundsätzlich auch als Band oder Schnur ausgebildet sein. Der Rückholabschnitt erstreckt sich vom distalen Ende des Behandlungskörpers ausgehend im Tragezustand des Behandlungskörpers aus der Körperhöhle heraus nach außen, so dass es möglich ist, den Rückholabschnitt an seinem freiliegenden Ende zu greifen und mit dem Rückholabschnitt den Behandlungskörper aus der Körperhöhle herauszuziehen. Vorzugsweise reicht der Rückholabschnitt im Behandlungs- bzw. Tragezustand des Behandlungskörpers um eine Länge von wenigstens 10 cm, vorzugsweise von wenigstens 20 cm oder mehr, aus der Körperhöhle nach außen. Der Rückholabschnitt kann vollständig aus Kunststoff, insbesondere einem elastischen Kunststoff, wie Silikon, Kautschuk oder auch Latex bestehen oder ein solches Material aufweisen. An seinem freien Ende kann der Rückholabschnitt einen Griffabschnitt aufweisen, der beispielsweise kugelförmig ausgebildet sein kann. Im Übrigen kann der Rückholabschnitt ein Antennenelement aufweisen für eine Datenübertragung zur Steuerschaltung, insbesondere drahtlos. Das Antennenelement kann für einen Empfang von Steuerdaten vorgesehen sein, die von einem externen Bediengerät, wie beispielsweise einem Smartphone, an das Antennenelement zur Steuerung der Bestrahlungseinrichtung übertragen werden können.

Der Behandlungskörper kann mehrere insbesondere miteinander über eine Schraubverbindung lösbar verbundene Gehäuseteile aufweise, insbesondere lediglich ein proximales Gehäuseteil und lediglich ein distales Gehäuseteil. Es können Mittel vorgesehen sein, um ein ungewolltes Lösen der Verbindung zwischen mehreren Gehäuseteilen zu verhindern, beispielsweise über eine Bajonettverbindung. Die Gehäuseteile können über ein Dichtmittel verbunden sein, um ein ungewolltes Eindringen von Flüssigkeit aus einem Körperhohlraum in das Innere des Behandlungskörpers zu verhindern.

Weist der Behandlungskörper ein proximales Gehäuseteil und ein distales Gehäuseteil auf, die miteinander insbesondere über eine Schraub- und/oder Bajonettverbindung gekoppelt sind, kann das proximale Gehäuseteil ein größeres Volumen aufweisen als das distale Gehäuseteil. In dem proximalen Gehäuseteil kann dann insbesondere die Bestrahlungseinrichtung aufgenommen sein.

An einem distalen Ende des Behandlungskörpers kann der Rückholabschnitt angeformt sein. Insbesondere kann der Rückholabschnitt Teil einer Ummantelung oder Umhüllung oder Beschichtung des Behandlungskörpers sein und aus einem elastischen Material, wie Silikon, Kautschuk oder Latex bestehen. Eine Ummantelung oder Umhüllung oder Beschichtung kann auf ein distales Gehäuseteil des Behandlungskörpers aufgezogen oder auf das Gehäuseteil aufgespritzt sein. Jedenfalls ist die Ummantelung oder Umhüllung oder Beschichtung fest mit dem Gehäuseteil verbunden, so dass es beim Herausziehen des Behandlungskörpers aus der Körperöffnung nicht zu einem Ablösen der Ummantelung oder Umhüllung oder Beschichtung vom Behandlungskörper kommen kann.

In einem Gehäuseteil, vorzugsweise in einem proximalen Gehäuseteil des Behandlungskörpers, kann wenigstens ein Leuchtmittel vorgesehen sein. Weiter vorzugsweise sind mehrere über den Umfang des proximalen Gehäuseteils gleichverteilt bzw. in Umfangsrichtung voneinander gleichbeabstandet angeordnete Leuchtmittel, insbesondere LEDs, vorgesehen.

Der Behandlungskörper kann, vorzugsweise vollflächig, aus einem lichtundurchlässigen Material bestehen und/oder eine lichtundurchlässige Oberfläche aufweisen und/oder es kann wenigstens eine Lichtöffnung vorgesehen sein für eine Lichtabgabe nach außen. Vorzugsweise ist im Bereich des proximalen Endes des Behandlungskörpers wenigstens eine Lichtöffnung vorgesehen, weiter vorzugsweise mehrere Lichtöffnungen, über die das Licht bei der Bestrahlung aus dem Behandlungskörper austritt und auf das umliegende Gewebe auftrifft. Nicht ausgeschlossen ist aber, dass das Gehäuse des Behandlungskörpers für eine Lichtabgabe nach außen vollständig oder auch lediglich bereichsweise aus einem lichtdurchlässigen Material besteht.

Sind mehrere Leuchtmittel vorgesehen, können auch mehrere Lichtöffnungen vorgesehen sein. Die Lichtöffnungen können voneinander beabstandet und voneinander getrennt sein durch Gehäusebereiche des Behandlungskörpers, die lichtundurchlässig sind. Bei einer bevorzugten Ausführungsform sind beispielsweise drei Lichtöffnungen vorgesehen und eine entsprechende Anzahl von LED-Leuchtmitteln, die einen Abstrahlwinkel von 120° aufweisen können. Entsprechend sind die Lichtöffnungen um einen Umfangswinkel von 120° voneinander beabstandet, bezogen auf die Mittelpunkte der Lichtöffnungen.

Jedem Leuchtmittel kann eine separate Lichtöffnung zugeordnet sein. Die Lichtöffnung ist vorzugsweise radial außenliegend vor einem zugeordneten Leuchtmittel vorgesehen.

Zum Verschließen der Lichtöffnung kann ein insbesondere scheibenartiges Verschlussteil aus einem lichtdurchlässigen Material vorgesehen sein. Vorzugsweise ist das Verschlussteil über ein Dichtmittel mit einem Gehäuseteil des Behandlungskörpers verbunden. Das Verschlussteil kann eine gerundete Oberfläche aufweisen und/oder angrenzende Oberflächen eines Gehäuseteils des Behandlungskörpers und des Verschlussteils können ausgefluchtet sein.

Um eine hohe Wertigkeit der Liebeskugel bzw. eine ästhetisch ansprechende äußere Erscheinungsform der Liebeskugel sicherzustellen, kann das Verschlussteil eine satinierte Oberfläche aufweisen. Durch die satinierte Oberfläche kommt es auch zur Lichtstreuung bei der Bestrahlung, was von Vorteil sein kann.

In einem proximalen Gehäuseteil des Behandlungskörpers kann ein Leiterplattenaufbau als Teil der Bestrahlungseinrichtung und wenigstens ein Leuchtmittel vorgesehen sein. Vorzugsweise ist ein distaler Gehäuseteil des Behandlungskörpers zur Aufnahme eines Speichers für elektrische Energie, insbesondere wenigstens einer Knopfzelle oder Batterie, ausgebildet und weist einen entsprechenden Aufnahmeraum auf. Der proximale Gehäuseteil und der distale Gehäuseteil können miteinander lösbar form- und/oder kraftschlüssig verbunden sein.

Der Behandlungskörper kann wenigstens ein Gehäuseteil aus Kunststoff, insbesondere aus Acrylnitril-Butadien-Styrol-Copolymer (ABS) oder Acrylnitril-Styrol-Acrylat-Copolymer (ASA) aufweisen. Weist der Behandlungskörper ein proximales Gehäuseteil und ein distales Gehäuseteil auf, die miteinander lösbar verbunden sind, bestehen vorzugsweise beide Gehäuseteile aus dem gleichen Kunststoff.

Das Verschlussteil zum Schließen einer Lichtöffnung kann aus einem transparenten Material, insbesondere aus Kunststoff oder Glas, bestehen, weiter insbesondere aus Polymethylmethacrylat (PMMA) oder ABS und/oder ein solches Material aufweisen. Die Oberfläche des Verschlussteils kann aufgeraut sein. Im Übrigen kann das Verschlussteil als Sammellinse oder Zerstreuungslinse ausgebildet sein und damit insbesondere eine Lupenwirkung erfüllen, was es zulässt, die Leistung der Lichtabgabequelle zu verringern.

Wenigstens ein Gehäuseteil des Behandlungskörpers, insbesondere ein proximales Gehäuseteil und ein distales Gehäuseteil, die miteinander lösbar verbunden sein können, weist außenseitig eine Beschichtung mit einem insbesondere elastischen Kunststoffmaterial, weiter insbesondere eine Silikon-, Kautschuk-, Latex- und/oder Polyurethanbeschichtung, auf. Besonders bevorzugt ist die Liebeskugel vollflächig, das heißt vorzugsweise der Behandlungskörper und der Rückholabschnitt, beschichtet, wobei verschiedene ummantelte oder beschichtete Gehäuseteile des Behandlungskörpers derart miteinander verbunden sein können, dass eine im Wesentlichen durchgehenden und flüssigkeitsdichte Außenschicht gebildet wird. Für die Verwendung in eine Körperöffnung kann die elastische Beschichtung seine Form anpassen und einen sicheren Sitz mit den umgebenden Körperwänden bilden.

Der Behandlungskörper der Liebeskugel kann außenseitig im Wesentlichen vollflächig beschichtet sein, mit Ausnahme von Lichtöffnungen, durch die das Licht bei der Bestrahlung nach außen dringt.

Der Rückholabschnitt kann im Wesentlichen langgestreckt und stabartig ausgebildet sein und an seinem dem Behandlungskörper zugewandten proximalen Ende einen Befestigungsabschnitt zur insbesondere form- und/oder stoffschlüssigen Befestigung mit dem Behandlungskörper aufweisen. Insbesondere besteht der Rückholabschnitt vollständig aus einem elastischen Material, wie Silikon, Latex oder Kautschuk. Der Befestigungsabschnitt kann in der Art einer Manschette ausgebildet sein, die eine Kavität zur Aufnahme eines distalen Gehäuseabschnitts des Behandlungskörpers bildet und/oder bereichsweise auf den distalen Gehäuseabschnitt aufgezogen oder aufgespritzt ist. Auch eine stoffschlüssige Verbindung zwischen dem Rückholabschnitt und einem Gehäuse des Behandlungskörpers durch Verkleben ist möglich.

Grundsätzlich kann der Behandlungskörper auch eine Beschichtung mit einem elastischen Material, wie Silikon, Latex oder Kautschuk aufweisen, wobei die Beschichtung einstückig in den Rückholabschnitt übergeht. In die Beschichtung können Verstärkungselemente eingebracht sein, um eine ausreichend hohe Zugfestigkeit des Rückholabschnitts zu gewährleisten, die erforderlich ist, um über den Rückholabschnitt den in die Körperöffnung eingesetzten Behandlungskörper herausziehen zu können. Die Beschichtung kann als elastische Schicht auf ein Gehäuseteil des Behandlungskörpers aufgespritzt oder aufgummiert sein.

Die Oberfläche des Behandlungskörpers kann mechanisch und/oder chemisch modifiziert sein und/oder es kann eine Oberflächenbeschichtung vorgesehen sein, um das Anhaften von wenigstens einem insbesondere wasserbasierten Photosensibilisator zu verbessern. Als Haftmittel kann beispielsweise Cellulose dienen. Wird ein Photosensibilisator auf den Behandlungskörper aufgebracht, kann dieser gefolgt von einer Beleuchtung eine fotodynamische Therapie unter Anwendung der erfindungsgemäßen Liebeskugel unterstützen. Der Sensibilisator kann sich aufgrund bestimmter Eigenschaften von Gewebeveränderungen mehr oder weniger selektiv in einer Gewebeveränderung anreichern. Nach einer gewissen Wartezeit wird die Gewebeveränderung dann mit Licht aus dem Behandlungskörper bestrahlt, wobei durch fotophysikalische Prozesse toxische Substanzen, vor allem reaktive Sauerstoffspezies, erzeugt werden können, die die Gewebeveränderung schädigen.

Um das umliegende Gewebe zu massieren, was Heilungsreaktionen durch Vibration insbesondere im vaginalen Gewebe auslösen bzw. unterstützen kann, kann die Liebeskugel auch einen Vibrationsmechanismus bzw. eine Schwingungserzeugungseinrichtung aufweisen, wie dies in der WO 2015/070242 A2 beschrieben ist.

Eine bevorzugte Ausführungsform einer Ausführungsform einer erfindungsgemäßen Liebeskugel wird nachfolgend anhand der Zeichnung beispielhaft beschrieben, wobei die Erfindung nicht auf die beschriebene Ausführungsform beschränkt ist. In der Zeichnung zeigen
- Fig. 1: eine perspektivische Ansicht einer erfindungsgemäßen Liebeskugel,
- Fig. 2: eine perspektivische Ansicht einer erfindungsgemäßen Liebeskugel, wobei äußere Gehäuseteile der Liebeskugel transparent dargestellt sind,
- Fig. 3: eine vergrößerte Teilansicht der Darstellung der Liebeskugel aus Fig. 2,
- Fig. 4: eine Explosionsdarstellung von Teilen der Liebeskugel aus Fig. 1 und
- Fig. 5: eine Darstellung der Bauteile einer erfindungsgemäßen Liebeskugel.

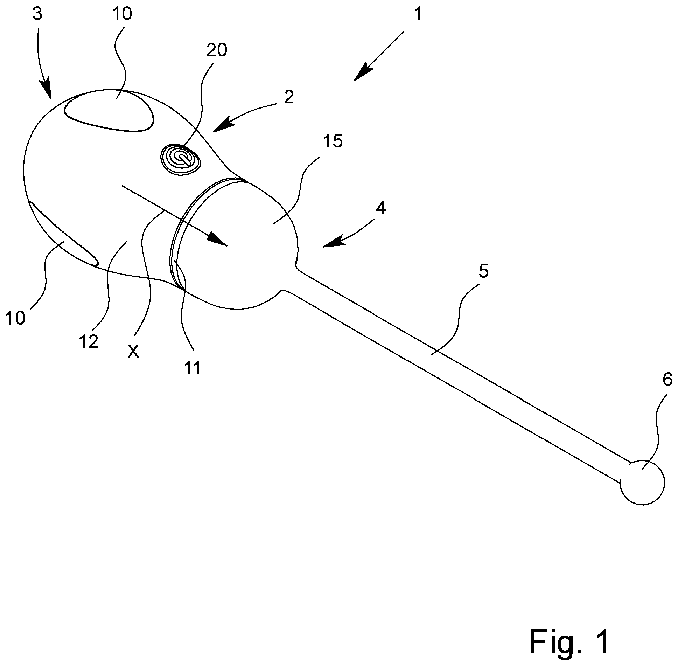

In den Fig. 1 bis 4 ist eine Liebeskugel 1 mit einem Behandlungskörper 2 zum Einführen in ein Vaginal- oder Rektallumen dargestellt und beschrieben. Der Behandlungskörper 2 weist ein proximales, körperzugewandtes Ende 3 und ein distales, körperabgewandtes Ende 4 auf. Am distalen Ende 4 ist ein Rückholabschnitt 5 zum Herausziehen des Behandlungskörpers 2 aus einem Körperlumen ausgebildet. Zum vereinfachten Herausziehen des Behandlungskörpers 2 weist der Rückholabschnitt 5 an seinem freien Ende einen kugelförmigen Griffabschnitt 6 auf.

Der Rückholabschnitt 5 weist eine hohe Elastizität und geringe Biegesteifigkeit auf, so dass der Rückholabschnitt 5 in der Art eines Bandes oder einer Schnur beim Tragen des Behandlungskörpers 2 nicht als störend wahrgenommen wird, wenn der Behandlungskörper 2 in ein Körperlumen eingeführt ist und der Rückholabschnitt 5 aus dem Körperlumen heraustritt.

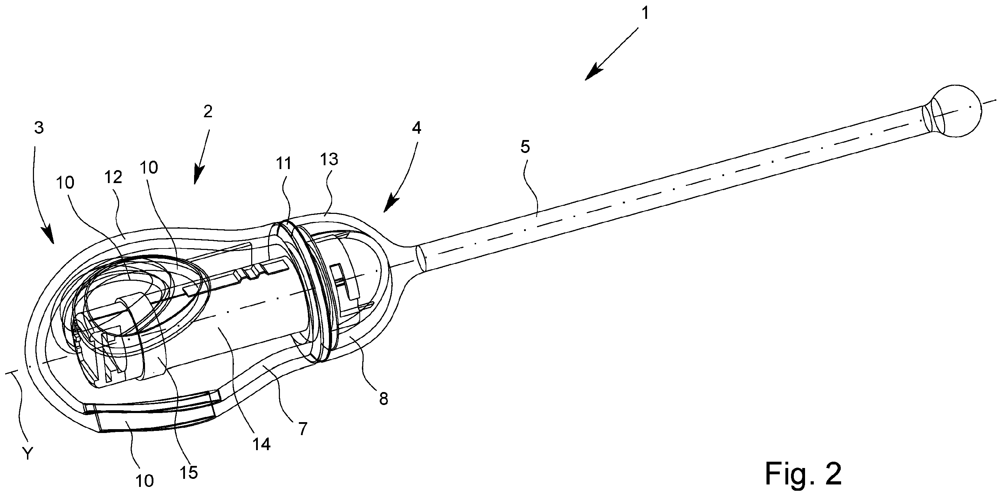

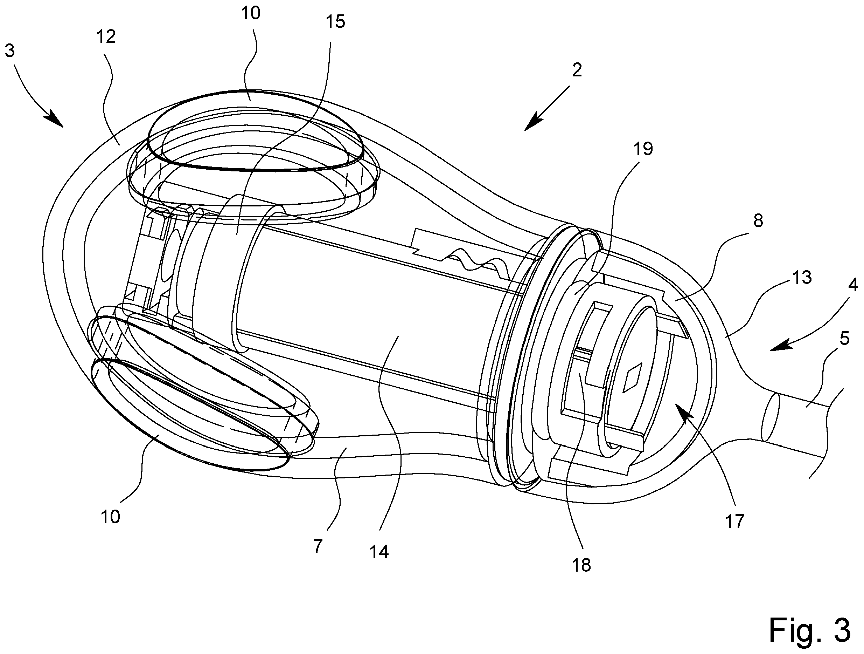

Wie sich weiter aus Fig. 1 ergibt, ist in einem Gehäuse des Behandlungskörpers 2, das bei der gezeigten Ausführungsform zweiteilig ist und ein proximales erstes Gehäuseteil 7 und ein distales zweites Gehäuseteil 8 umfasst, eine Mehrzahl von Lichtöffnungen 9 vorgesehen, die mit scheibenartigen Verschlusselementen 10 geschlossen sind. Die Gehäuseteile 7, 8 sind in den Fig. 2 und 3 dargestellt und können aus vorzugsweise lichtundurchlässigem Hartkunststoff bestehen, beispielsweise ABS.

Das proximale Gehäuseteil 7 und das distale Gehäuseteil 8 sind lösbar und form-, kraft- und/oder stoffschlüssig miteinander verbunden. Bei der vorliegenden Ausführungsform sind die Gehäuseteile 7, 8 über eine Bajonettverbindung oder gegebenenfalls auch über eine reine Steck- oder Drehverbindung lösbar miteinander verbunden, so dass es möglich ist, die Gehäuseteile 7, 8 bedarfsweise für einen Batteriewechsel voneinander zu trennen. Zwischen den Gehäuseteilen 7, 8 kann ein Ringelement 11 vorgesehen, dass auch als Dichtring ausgebildet sein kann. Das Ringelement 11 kann jedoch auch lediglich dekorativen Zwecken dienen und beispielsweise aus Metall bestehen.

Wie sich weiter aus den Fig. 1 bis 3 ergibt, weist das proximale Gehäuseteil 7 eine äußere Ummantelung 12 und das distale Gehäuseteil 8 eine äußere Ummantelung 13 auf. Die Ummantelung 12, 13 besteht aus Silikon oder einem anderen elastischen Kunststoffmaterial. Mit Ausnahme der Lichtöffnungen 9 sind die Gehäuseteile 7, 8 im Wesentlichen vollflächig außenseitig beschichtet bzw. von der Ummantelung 12, 13 umgeben. Die Ummantelungen 12, 13 können stirnseitig direkt gegeneinander anliegen, so dass ein dichtender Kontakt hergestellt wird. Grundsätzlich kann aber auch vorgesehen sein, dass, so wie in den Fign. 1 bis 3 gezeigt, das Ringelement 11 nach außen sichtbar und nicht von den Ummantelungen 12, 13 ummantelt ist. Die Ummantelungen 12, 13 können dann stirnseitig gegen das Ringelement 11 anliegen, so dass eine hohe Dichtheit des Inneren der Liebeskugel 1 gegenüber der Umgebung sichergestellt ist. Es wird eine im Wesentlichen nach außen geschlossene und flüssigkeitsdichte Oberfläche der Liebeskugel 1 ausgebildet, was das Eindringen von Körperflüssigkeit in das Innere des Behandlungskörpers 2 verhindert.

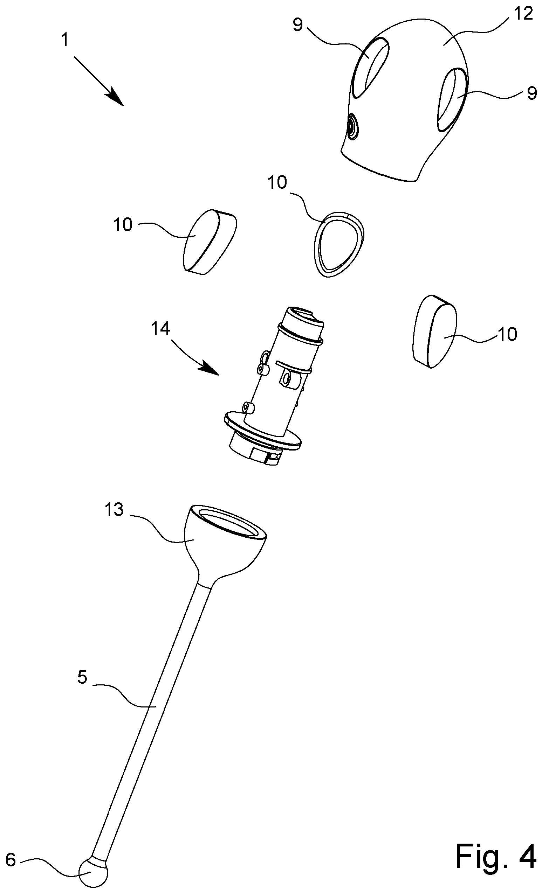

Bei der gezeigten Ausführungsform ist der Behandlungskörper 2 im Wesentlichen vollflächig außenseitig ummantelt und/oder beschichtet. Ausgenommen sind lediglich die Lichtöffnungen 9, was insbesondere Fig. 4 zeigt. Damit weist der Behandlungskörper 2 mit Ausnahme der Lichtöffnungen 9 eine im Wesentlichen geschlossene, integrale Oberfläche auf. Insbesondere weist der Behandlungskörper 2 keine Hinterschneidungen oder scharfen Kanten auf, was die Besiedlung mit Mikroorganismen unterstützen und die Reinigung der Oberfläche des Behandlungskörpers 2 erschweren könnte.

Insbesondere ist die Oberfläche des Behandlungskörpers 2 vollflächig flüssigkeitsdicht gegenüber der Umgebung ausgeführt, was insbesondere auch die Verbindungsbereiche zwischen den Lichtöffnungen 9 und den Verschlusselementen 10 einerseits und zwischen dem proximalen Gehäuseteil 7 und dem distalen Gehäuseteil 8 mit dem daran einstückig angeformten Rückholabschnitt 5 betrifft.

Der Behandlungskörper 2 weist die Form eines Rotationskörpers auf, der sich in distaler Richtung X (Fig. 1) verjüngt. Die Form des Behandlungskörpers 2 kann als "birnenförmig" oder "konusförmig" beschrieben werden, wobei der Behandlungskörper einen kreisförmigen oder elliptischen Querschnitt aufweisen kann und länglich ausgebildet ist.

Die Form des Behandlungskörpers 2 ist vorgegeben durch die Form der Gehäuseteile 7, 8, wobei das proximale Gehäuseteil 7 gebildet wird durch einen halbkugelförmigen Abschnitt mit größerem Durchmesser im Bereich des proximalen Endes 3 des Behandlungskörpers 2, der in einen taillierten mittleren Abschnitt übergeht. Das distale Gehäuseteil 8 ist halbkugelförmig ausgebildet mit kleinerem Durchmesser und grenzt an den taillierten mittleren Abschnitt des proximalen Gehäuseteils 7 an.

Wie nachfolgend beschrieben wird, weist der Behandlungskörper 2 eine integrierte Bestrahlungseinrichtung mit mehreren Lichtemittern auf, wobei bei der gezeigten Ausführungsform in dem Gehäuse des Behandlungskörpers 2 beispielsweise drei Lichtöffnungen 9 vorgesehen sind, die benachbart zum proximalen Ende 3 des Behandlungskörpers 2 angeordnet und äquidistant über die Umfangsfläche des Behandlungskörpers 2 verteilt sind.

Das Gehäuse des Behandlungskörpers 2 besteht vorzugsweise aus einem lichtundurchlässigen Material und/oder weist eine lichtundurchlässige Beschichtung auf, so dass lediglich über die Lichtöffnungen 9 Licht nach außen dringen kann.

Wie sich aus den Fig. 2 bis 4 ergibt, ist eine Technikeinheit 14 in das proximale Gehäuseteil 7 des Behandlungskörpers 2 integriert, die eine nicht im Einzelnen dargestellte Bestrahlungseinrichtung mit LED-Leuchtmitteln umfasst, wobei die LED-Leuchtmittel auf einem ringförmigen LED-Board 15 angeordnet und in Umfangsrichtung gleichweit voneinander beabstandet um die Längsachse Y der Liebeskugel 1 verteilt angeordnet sein können. Die LEDs können jeweils einen Abstrahlwinkel von 120° aufweisen. Die Hauptstrahlungsrichtung der LEDs ist hierbei radial nach außen gerichtet, wobei jeder LED eine Lichtöffnung 9 mit in die Lichtöffnung 9 eingesetztem Verschlusselement 10 zugeordnet ist.

Die Bestrahlungseinrichtung ist konfiguriert zur Emission von Licht mit einer Wellenlänge zwischen 600 und 750 nm, vorzugsweise zwischen 620 und 640 nm, weiter vorzugsweise von 630 nm, bei eine möglichen Peakabweichung von ± 3 nm. Im Übrigen ist die Bestrahlungseinrichtung konfiguriert zur Beleuchtung mit einer Strahlungsstärke zwischen 0,05 und 20 mW/cm², vorzugsweise zwischen 0,1 und 1,5 mW/cm², weiter vorzugsweise zwischen 0,25 und 1,0 mW/cm². Die maximale Bestrahlungsdauer mit der Bestrahlungseinrichtung beträgt vorzugsweise weniger als 30 Minuten, weiter vorzugsweise kleiner oder gleich 20 Minuten.

Durch die Emission von Licht mit einer bestimmten Wellenlänge und einer bestimmten maximalen Bestrahlungsstärke lässt sich die Liebeskugel 1 in vorteilhafter Weise einsetzen zur Gewebeverjüngung durch fotodynamische Therapie in Körperhohlräumen, mit oder ohne Verwendung von Photosensibilisatoren, insbesondere zur vaginalen Verjüngung durch Anregung der Kollagenneubildung und/oder der Gewebeerneuerung, und/oder zur Behandlung von Gewebeveränderungen in Körperhohlräumen, insbesondere zur Behandlung der zervikalen Dysplasie, und/oder zur Behandlung von insbesondere bakteriellen oder Pilzinfektionen.

Im Übrigen umfasst die Technikeinheit 14 eine nicht im Einzelnen gekennzeichnete Steuerschaltung mit einem Leiterplattenaufbau zur Steuerung oder Regelung der Bestrahlungseinrichtung.

Das Verschlussteil 10 besteht aus einem lichtdurchlässigen Material und kann, vorzugsweise, eine satinierte Oberfläche aufweisen. Damit ist eine Lichtemission aus dem Bestrahlungskörper 2 und die Bestrahlung des den Behandlungskörper 2 im Tragezustand bzw. während der therapeutischen Behandlung umgebenden Körpergewebes möglich. Das Verschlussteil 10 kann auch als Sammellinse oder Zerstreuungslinse ausgebildet sein, um durchgehendes Licht zu brechen und zur Mitte abzulenken oder nach außen zu streuen. Auch kann das Verschlusselement in der Art eines optischen Filters wirken und Strahlung nach bestimmten Kriterien, beispielsweise Wellenlänge, Polarisationszustand oder Strahlungsrichtung, selektieren. Dies gilt insbesondere dann, wenn an der Stelle von LEDs andere Leuchtmittel eingesetzt werden, was nicht ausgeschlossen ist.

Wie sich insbesondere aus Fig. 2 und Fig. 3 ergibt, ist im Inneren des distalen Gehäuseteils 8 ein Aufnahmeraum 17 zur Aufnahme einer Batterie für die Stromversorgung der Bestrahlungseinrichtung vorgesehen. Der Aufnahmeraum 17 kann ein Batteriefach zur Aufnahme insbesondere einer Knopfbatterie bilden. Über eine entsprechende Verbindungsgeometrie 18 am distalen Ende der Technikeinheit 14 lässt sich diese mit dem distalen Gehäuseteil 8 mittelbar oder unmittelbar form- und/oder kraftschlüssig verbinden, insbesondere über eine Bajonettverbindung. Für eine stabile Verbindung kann ein Dichtring 19 vorgesehen sein.

Zum Ein- und Ausschalten der Liebeskugel 1 ist ein An-/Ausschalter 20 vorgesehen, der flüssigkeitsdicht in die äußere Ummantelung 12 des proximalen Gehäuseteils 7 eingesetzt ist.

Nicht gezeigt ist, dass ein Aufnahmeraum 17 beispielsweise innerhalb des distalen Gehäuseteils 8 zur Aufnahme einer austauschbaren Beschwerungsmasse vorgesehen sein kann, wobei durch unterschiedlich schwere Beschwerungsmassen ein unterschiedliches Gewicht der Liebeskugel 1 und damit eine unterschiedliche therapeutische Wirkung bei der Anwendung der Liebeskugel 1 als Mittel zur therapeutischen Behandlung der Beckenbodenmuskulatur erreicht wird. Grundsätzlich kann auch vorgesehen sein, dass die Gehäuseteile 7, 8 aus einem schweren Material bestehen, beispielsweise aus Metall, um ein höheres Gewicht der Liebeskugel 1 zu erreichen.

Der Behandlungskörper 2 kann ein Gesamtgewicht von wenigstens 30 g, vorzugsweise von wenigstens 40 g, weiter vorzugsweise von 50 g, besonders bevorzugt von 60 g, und/oder von weniger als 100 g aufweisen, bezogen auf den Funktionszustand der Liebeskugel 1, wenn wenigstens eine Batterie zur Stromversorgung der Bestrahlungseinrichtung und/oder ein Beschwerungsgewicht in den Behandlungskörper 2 eingesetzt ist.

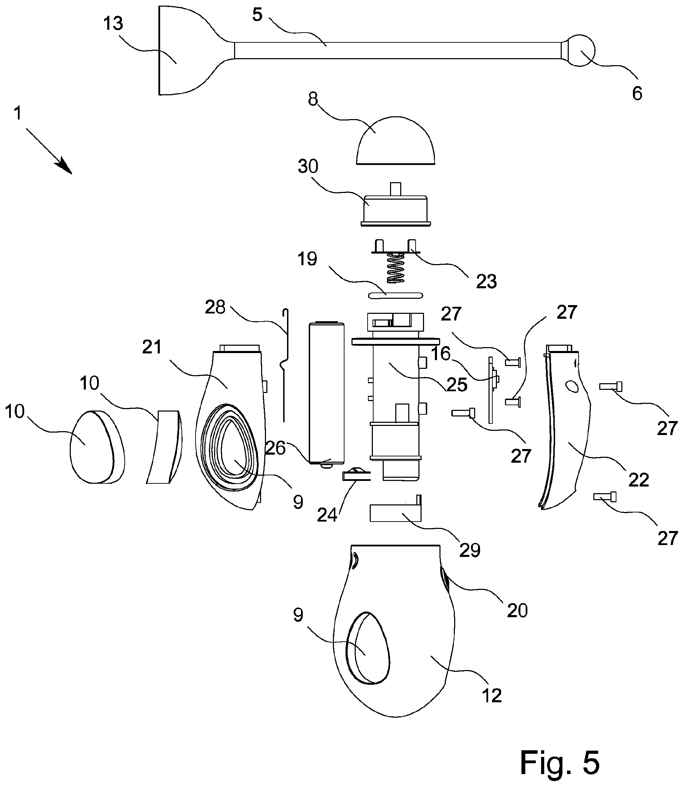

Fig. 5 zeigt eine Ansicht der möglichen Bauteile einer Liebeskugel 1. Bei den Bauteilen der Liebeskugel 1 handelt es sich um den Rückholabschnitt 5 mit Ummantelung 13 und Griffabschnitt 6, zwei Gehäuseschalen 21, 22 des proximalen Gehäuseteils 7, die Ummantelung 12 des proximalen Gehäuseteils 7, das zweite distale Gehäuseteil 8 bzw. eine Endkappe, die Verschlusselemente 10 für die Lichtöffnungen 9 und den Dichtring 19.

Im Übrigen weist die Liebeskugel 1 ein Federkontaktelement 23 und eine Kontaktplatte 24 auf, um wenigstens eine in einen Batteriehalter 25 eingesetzte Batterie 26 elektrisch zu kontaktieren. Das Federkontaktelement 23 kontaktiert den Minuspol der Batterie 26 und die Kontaktplatte 24 den Pluspol der Batterie 26, so dass eine Stromversorgung sichergestellt ist.

Zur Verbindung der Bauteile miteinander sind mehrere Schrauben 27 als Verbindungsmittel vorgesehen.

Ferner umfasst die Liebeskugel 1 ein weiteres Kontaktelement 28, einen Leiterplattenaufbau 16 für die Hauptsteuerung der Liebeskugel 1 und einen flexiblen LED-Leiterplattenaufbau 29.

Ein Herzstück 30 ist als Halter für das Federkontaktelement 23 vorgesehen. Im Montagezustand ist das Herzstück 30 in dem distalen Gehäuseteil 8 aufgenommen.

Im Übrigen kann vorgesehen sein, dass das Herzstück 30 mit der Endkappe 8 zusammenwirkt, was ein Eindrehen/Ausdrehen des Batteriehalters 25 ermöglicht.

Es versteht sich, dass die vorgenannten Ausführungsformen der Liebeskugel 1 bedarfsweise miteinander kombiniert werden können, auch wenn dies nicht im Einzelnen ausdrücklich beschrieben ist.

Der Anteil der durch die Lichtöffnungen 9 gebildeten Gesamtöffnungsfläche der Liebeskugel 1 für über die Lichtöffnungen 9 austretende Lichtstrahlung an der Gesamtoberfläche der Ummantelung 12 des proximalen Gehäuseteils 7 kann zwischen 5 bis 20 %, vorzugsweise zwischen 10 und 15 %, betragen. Im Rahmen der beschriebenen Erfindung durchgeführte Vergleichsversuche haben ergeben, dass bei den zuvor genannten Anteilen eine ausreichende Lichtemission einerseits bei hoher Stabilität des proximalen Gehäuseteils 7 andererseits gewährleistet ist.

### Bezugszeichenliste:

- 1: Liebeskugel
- 2: Behandlungskörper
- 3: proximales Ende
- 4: distales Ende
- 5: Rückholabschnitt
- 6: Griffabschnitt
- 7: proximales Gehäuseteil
- 8: distales Gehäuseteil
- 9: Lichtöffnung
- 10: Verschlusselement
- 11: Ringelement
- 12: Ummantelung
- 13: Ummantelung
- 14: Technikeinheit
- 15: LED-Board
- 16: Leiterplattenaufbau
- 17: Aufnahmeraum
- 18: Verbindungsgeometrie
- 19: Dichtring
- 20: An-/Ausschalter
- 21: Gehäuseschale
- 22: Gehäuseschale
- 23: Federkontaktelement
- 24: Kontaktplatte
- 25: Batteriehalter
- 26: Batterie
- 27: Schraube
- 28: Kontaktelement
- 29: Leiterplattenaufbau
- 30: Herzstück

## Patentansprüche

1. Liebeskugel (1) mit einem Behandlungskörper (2) zum Einführen in ein Vaginal- oder Rektallumen, mit einer in den Behandlungskörper (2) integrierten Bestrahlungseinrichtung mit wenigstens einem Lichtemitter, mit einer in den Behandlungskörper (2) integrierten Stromquelle zur Stromversorgung der Bestrahlungseinrichtung und mit einer in den Behandlungskörper (2) integrierten Steuerschaltung zur Steuerung der Bestrahlungseinrichtung, wobei die Bestrahlungseinrichtung konfiguriert ist zur Emission von Licht mit einer Wellenlänge zwischen 600 nm und 750 nm und **dadurch gekennzeichnet, dass** die Bestrahlungseinrichtung Bestrahlung mit einer maximalen Bestrahlungsstärke zwischen 0,1 mW/cm² und 1,5 mW/cm² und wobei die Steuerschaltung zum automatischen Abschalten der Bestrahlung bei Erreichen einer Bestrahlungsenergie von weniger als 5 J und/oder nach einer Bestrahlungsdauer von kleiner oder gleich 60 min ausgebildet ist.

2. Liebeskugel (1) nach Anspruch 1, wobei die Bestrahlungseinrichtung konfiguriert ist zur Emission von Licht mit einer Wellenlänge zwischen 620 nm und 640 nm, vorzugsweise von 630 nm und/oder dass die Bestrahlungseinrichtung konfiguriert ist zur Beleuchtung mit einer Bestrahlungsstärke zwischen 0,25 mW/cm² und 1,0 mW/cm².

3. Liebeskugel (1) nach einem der vorhergehenden Ansprüche, wobei die Steuerschaltung zum automatischen Abschalten der Bestrahlung bei Erreichen einer Bestrahlungsenergie von weniger als 2,0 J, besonders bevorzugt von 0,5 J bis 1,5 J, und/oder nach einer Bestrahlungsdauer von kleiner oder gleich 30 min, weiter vorzugsweise von kleiner oder gleich 20 min, ausgebildet ist.

4. Liebeskugel (1) nach einem der vorhergehenden Ansprüche, wobei der Behandlungskörper (2) ein Gewicht von wenigstens 30 g, vorzugsweise von wenigstens 40 g, weiter vorzugsweise von 50 g, besonders bevorzugt von 60 g, und/oder von weniger als 100 g aufweist und/oder dass wenigstens ein in den Behandlungskörper (2) integrierter Aufnahmeraum zur Aufnahme wenigstens einer austauschbaren Beschwerungsmasse vorgesehen ist, vorzugsweise zur Aufnahme unterschiedlich schwerer Beschwerungsmassen.

5. Liebeskugel (1) nach Anspruch 4, wobei wenigstens eine austauschbare Beschwerungsmasse vorgesehen ist und dass, vorzugsweise, die Beschwerungsmasse ein Gewicht zwischen 5 g und 15 g, vorzugsweise 10 g, aufweist.

6. Liebeskugel (1) nach einem der vorhergehenden Ansprüche, wobei eine Temperaturmesseinrichtung zur Erfassung der Oberflächentemperatur an der Außenfläche des Behandlungskörpers (2) vorgesehen ist und dass, vorzugsweise, die Steuerschaltung zum automatischen Abschalten der Bestrahlung bei Erreichen einer Oberflächentemperatur von kleiner oder gleich 45 °C, vorzugsweise von kleiner oder gleich 42 °C, weiter vorzugsweise von kleiner oder gleich 40 °C, ausgebildet ist und/oder dass die Oberflächentemperatur des Behandlungskörpers (2) bei unterbrechungsloser Bestrahlung mit einer maximalen Bestrahlungsstärke über einen Zeitraum von weniger als 60 min, vorzugsweise von weniger als 30 min, weiter vorzugsweise von weniger als 20 min., weniger als 45 °C, vorzugsweise weniger als 42 °C, weiter vorzugsweise weniger als 40 °C, beträgt.

7. Liebeskugel (1) nach einem der vorhergehenden Ansprüche, wobei die Bestrahlungseinrichtung Licht kontinuierlich emittiert oder gepulst betrieben wird.

8. Liebeskugel (1) nach einem der vorhergehenden Ansprüche, wobei die Steuerschaltung konfiguriert ist zum automatischen Abschalten der Bestrahlung nach Erreichen einer Bestrahlungsdauer von weniger als 30 min, weiter vorzugsweise von weniger als 20 min, und/oder bei Erreichen einer emittierten Bestrahlungsenergie von weniger als 37 J/cm², vorzugsweise von weniger als 5,0 J/cm², weiter vorzugsweise von weniger als 2,0 J/cm², besonders bevorzugt von 0,5 J/cm² bis 1,5 J/cm².

9. Liebeskugel (1) nach einem der vorhergehenden Ansprüche, wobei eine Kühleinrichtung zur Kühlung des Behandlungskörpers (2) vorgesehen ist.

10. Liebeskugel (1) nach einem der vorhergehenden Ansprüche, wobei die Beleuchtungseinrichtung mehrere LEDs aufweist, wobei, vorzugsweise, eine Anordnung der LEDs und/oder ein Abstrahlwinkel der LEDs vorgesehen ist, der eine vollumfängliche Ausleuchtung ermöglicht.

11. Liebeskugel (1) nach einem der vorhergehenden Ansprüche, wobei der Behandlungskörper (2) die Form eines Rotationskörpers aufweist und/oder dass der Behandlungskörper (2) eine im Wesentlichen geschlossene, integrale Oberfläche aufweist.

12. Liebeskugel (1) nach einem der vorhergehenden Ansprüche, wobei der Behandlungskörper (2) mehrere miteinander insbesondere über eine Schraubverbindung verbundene Gehäuseteile (7, 8) aufweist, insbesondere ein proximales Gehäuseteil (7) und ein distales Gehäuseteil (8) und/oder dass in einem Gehäuseteil (7), vorzugsweise in einem proximalen Gehäuseteil (7), des Behandlungskörpers (2), wenigstens ein Leuchtmittel, vorzugsweise mehrere über den Umfang des proximalen Gehäuseteils gleichverteilt angeordnete Leuchtmittel, insbesondere LEDs, vorgesehen sind.

13. Liebeskugel (1) nach einem der vorhergehenden Ansprüche, wobei ein Gehäuse (7, 8) des Behandlungskörpers (2) aus einem lichtundurchlässigen Material besteht und/oder eine lichtundurchlässige Oberfläche aufweist und/oder dass in dem Gehäuse (7) wenigstens eine Lichtöffnung (9) vorgesehen ist für eine Lichtabgabe nach außen und/oder dass mehrere Leuchtmittel und mehrere Lichtöffnungen (9) vorgesehen sind, wobei, vorzugsweise, dass jedem Leuchtmittel eine separate Lichtöffnung (9) zugeordnet ist.

14. Liebeskugel (1) nach Anspruch 13, wobei die Lichtöffnung (9) mit einem insbesondere scheibenartigen Verschlusselement (10) aus einem lichtdurchlässigen Material verschlossen ist, wobei, vorzugsweise, das Verschlusselement (10) eine satinierte Oberfläche aufweist und/oder dass das Verschlusselement (10) aus einem transparenten Material, insbesondere aus Kunststoff, weiter insbesondere aus ABS, besteht und/oder ein solches Material aufweist und/oder dass das Verschlusselement (10) als Sammellinse oder Zerstreuungslinse ausgebildet ist.

15. Liebeskugel (1) nach einem der vorhergehenden Ansprüche, wobei wenigstens ein Gehäuseteil (7, 8) des Behandlungskörpers (2), insbesondere ein proximales Gehäuseteil (7) und ein distales Gehäuseteil (8), außenseitig eine Ummantelung (12, 13) und/oder Beschichtung aus einem insbesondere elastischen Kunststoffmaterial aufweist, insbesondere eine Silikon-, Kautschuk-, Latex- und/oder Polyurethanummantelung und/oder -beschichtung und/oder dass eine Ummantelung (13) und/oder Beschichtung am distalen Ende (4) des Behandlungskörpers (2) und ein Rückholabschnitt (5) einstückig ausgebildet sind und/oder dass der Behandlungskörper (2) im Wesentlichen vollflächig außenseitig ummantelt und/oder beschichtet ist.

## Claims

1. A love ball (1) with a treatment body (2) for insertion into a vaginal or rectal cavity, with an irradiation device integrated into the treatment body (2) with at least one light emitter, with a power source integrated into the treatment body (2) for power supply of the irradiation device and with a control circuit integrated into the treatment body (2) for control of the irradiation device, wherein the irradiation device is configured for emission of light with a wavelength between 600 nm and 750 nm and **characterized in that** the irradiation device is configured for irradiation with a maximum irradiation intensity between 0.1 mW/cm² and 1.5 mW/cm² and wherein the control circuit is configured for automatic switching off of the irradiation on reaching an irradiation energy of less than 5 J and/or after an irradiation duration of less than or equal to 60 min.

2. The love ball (1) according to claim 1, wherein the irradiation device is configured for emission of light with a wavelength between 620 nm and 640 nm, preferably of 630 nm and/or that the irradiation device is configured for illumination with an irradiation intensity between 0.25 mW/cm² and 1.0 mW/cm².

3. The love ball (1) according to one of the preceding claims, wherein the control circuit is configured for automatic switching off of the irradiation on reaching an irradiation energy of less than 2.0 J, particularly preferably of 0.5 J to 1.5 J, and/or after an irradiation duration of less than or equal to 30 min, further preferably of less than or equal to 20 min.

4. The love ball (1) according to one of the preceding claims, wherein the treatment body (2) has a weight of at least 30 g, preferably of at least 40 g, further preferably of 50 g, particularly preferably of 60 g, and/or of less than 100 g and/or that at least one receiving space integrated into the treatment body (2) is provided for receiving at least one exchangeable weighting mass, preferably for receiving weighting masses of different weights.

5. The love ball (1) according to claim 4, wherein at least one exchangeable weighting mass is provided and that, preferably, the weighting mass has a weight between 5 g and 15 g, preferably 10 g.

6. The love ball (1) according to one of the preceding claims, wherein a temperature measuring device for detecting the surface temperature is provided on the outer surface of the treatment body (2) and that, preferably, the control circuit is configured for automatic switching off of the irradiation on reaching a surface temperature of less than or equal to 45 °C, preferably of less than or equal to 42 °C, further preferably of less than or equal to 40 °C and/or that the surface temperature of the treatment body (2) on uninterrupted irradiation with a maximum irradiation intensity over a period of less than 60 min, preferably of less than 30 min, further preferably of less than 20 min, is less than 45 °C, preferably less than 42 °C, further preferably less than 40 °C.

7. The love ball (1) according to one of the preceding claims, wherein the irradiation device emits light continuously or is operated in a pulsed manner.

8. The love ball (1) according to one of the preceding claims, wherein the control circuit is configured for automatic switching off of the irradiation on reaching an irradiation duration of less than 30 min, further preferably of less than 20 min, and/or on reaching an emitted irradiation energy of less than 37 J/cm² , preferably of less than 5.0 J/cm² , further preferably of less than 2.0 J/cm² , particularly preferably of 0.5 J/cm² to 1.5 J/cm² .

9. The love ball (1) according to one of the preceding claims, wherein a cooling device for cooling the treatment body (2) is provided.

10. The love ball (1) according to one of the preceding claims, wherein the illumination device has a plurality of LEDs, wherein, preferably, an arrangement of the LEDs and/or an emission angle of the LEDs is provided, which enables a full-circumference illumination.

11. The love ball (1) according to one of the preceding claims, wherein the treatment body (2) has the shape of a rotational body and/or that the treatment body (2) has a substantially closed, integral surface.

12. The love ball (1) according to one of the preceding claims, wherein the treatment body (2) has a plurality of housing parts (7, 8) connected to one another in particular via a screw connection, in particular a proximal housing part (7) and a distal housing part (8) and/or that in a housing part (7), preferably in a proximal housing part (7), of the treatment body (2), at least one lighting means, preferably a plurality of lighting means, in particular LEDs, arranged uniformly distributed over the circumference of the proximal housing part are provided.

13. The love ball (1) according to one of the preceding claims, wherein a housing (7, 8) of the treatment body (2) consists of a light-impermeable material and/or has a light-impermeable surface and/or that in the housing (7) at least one light opening (9) is provided for a light emission to the outside and/or that a plurality of lighting means and a plurality of light openings (9) are provided, wherein, preferably, a separate light opening (9) is assigned to each lighting means.

14. The love ball (1) according to claim 13, wherein the light opening (9) is closed with an in particular disk-like closure element (10) made of a light-permeable material, wherein, preferably, the closure element (10) has a satinized surface and/or that the closure element (10) consists of a transparent material, in particular of plastic, further in particular of ABS, and/or has such a material and/or that the closure element (10) is configured as a converging lens or diverging lens.

15. The love ball (1) according to one of the preceding claims, wherein at least one housing part (7, 8) of the treatment body (2), in particular a proximal housing part (7) and a distal housing part (8), has on the outside a sheathing (12, 13) and/or coating made of an in particular elastic plastic material, in particular a silicone, rubber, latex and/or polyurethane sheathing and/or coating and/or that a sheathing (13) and/or coating at the distal end (4) of the treatment body (2) and a return section (5) are configured in one piece and/or that the treatment body (2) is sheathed and/or coated on the outside substantially over the entire surface.

## Revendications

1. Boule d'amour (1) avec un corps de traitement (2) pour l'introduction dans un système vaginal ou rectal, avec un dispositif d'irradiation intégré dans le corps de traitement (2) avec au moins un émetteur de lumière, avec une source de courant intégrée dans le corps de traitement (2) pour l'alimentation en courant du dispositif d'irradiation et avec un circuit de commande intégré dans le corps de traitement (2) pour la commande du dispositif d'irradiation, dans laquelle le dispositif d'irradiation est configuré pour l'émission de lumière avec une longueur d'onde comprise entre 600 nm et 750 nm et **caractérisée en ce que** le dispositif d'irradiation est conçu pour l'irradiation avec une intensité d'irradiation maximale comprise entre 0,1 mW/cm² et 1,5 mW/cm² et dans laquelle le circuit de commande est conçu pour l'arrêt automatique de l'irradiation lorsqu'une énergie d'irradiation inférieure à 5 Jet/ou après une durée d'irradiation inférieure ou égale à 60 minutes est atteinte.

2. Boule d'amour (1) selon la revendication 1, dans laquelle le dispositif d'irradiation est configuré pour l'émission de lumière avec une longueur d'onde comprise entre 620 nm et 640 nm, de préférence de 630 nm et/ou en ce que le dispositif d'irradiation est configuré pour l'éclairage avec une intensité d'irradiation comprise entre 0,25 mW/cm² et 1,0 mW/cm² .

3. Boule d'amour (1) selon l'une quelconque des revendications précédentes, dans laquelle le circuit de commande est conçu pour l'arrêt automatique de l'irradiation lorsqu'une énergie d'irradiation inférieure à 2,0 J, de manière particulièrement préférée de 0,5 J à 1,5 J, et/ou après une durée d'irradiation inférieure ou égale à 30 minutes, de manière davantage préférée inférieure ou égale à 20 minutes, est atteinte.

4. Boule d'amour (1) selon l'une quelconque des revendications précédentes, dans laquelle le corps de traitement (2) présente un poids d'au moins 30 g, de préférence d'au moins 40 g, de manière davantage préférée de 50 g, de manière particulièrement préférée de 60 g, et/ou inférieur à 100 g et/ou en ce qu'au moins un espace de réception intégré dans le corps de traitement (2) est prévu pour la réception d'au moins une masse de lestage échangeable, de préférence pour la réception de masses de lestage de poids différents.

5. Boule d'amour (1) selon la revendication 4, dans laquelle au moins une masse de lestage échangeable est prévue et en ce que, de préférence, la masse de lestage présente un poids compris entre 5 g et 15 g, de préférence 10 g.

6. Boule d'amour (1) selon l'une quelconque des revendications précédentes, dans laquelle un dispositif de mesure de température est prévu pour la détection de la température de surface sur la surface extérieure du corps de traitement (2) et en ce que, de préférence, le circuit de commande est conçu pour l'arrêt automatique de l'irradiation lorsqu'une température de surface inférieure ou égale à 45 °C, de préférence inférieure ou égale à 42 °C, de manière davantage préférée inférieure ou égale à 40 °C, est atteinte et/ou en ce que la température de surface du corps de traitement (2) en cas d'irradiation ininterrompue avec une intensité d'irradiation maximale sur une durée inférieure à 60 minutes, de préférence inférieure à 30 minutes, de manière davantage préférée inférieure à 20 minutes, est inférieure à 45 °C, de préférence inférieure à 42 °C, de manière davantage préférée inférieure à 40 °C.

7. Boule d'amour (1) selon l'une quelconque des revendications précédentes, dans laquelle le dispositif d'irradiation émet de la lumière en continu ou fonctionne de manière pulsée.

8. Boule d'amour (1) selon l'une quelconque des revendications précédentes, dans laquelle le circuit de commande est configuré pour l'arrêt automatique de l'irradiation après qu'une durée d'irradiation inférieure à 30 minutes, de manière davantage préférée inférieure à 20 minutes, est atteinte et/ou lorsqu'une énergie d'irradiation émise inférieure à 37 J/cm², de préférence inférieure à 5,0 J/cm², de manière davantage préférée inférieure à 2,0 J/cm2, de manière particulièrement préférée de 0,5 J/cm² à 1,5 J/cm², est atteinte.

9. Boule d'amour (1) selon l'une quelconque des revendications précédentes, dans laquelle un dispositif de refroidissement est prévu pour le refroidissement du corps de traitement (2).

10. Boule d'amour (1) selon l'une quelconque des revendications précédentes, dans laquelle le dispositif d'éclairage présente plusieurs LED, dans laquelle, de préférence, un agencement des LED et/ou un angle de rayonnement des LED est prévu, qui permet un éclairage sur toute la périphérie.

11. Boule d'amour (1) selon l'une quelconque des revendications précédentes, dans laquelle le corps de traitement (2) présente la forme d'un corps de révolution et/ou en ce que le corps de traitement (2) présente une surface intégrale sensiblement fermée.

12. Boule d'amour (1) selon l'une quelconque des revendications précédentes, dans laquelle le corps de traitement (2) présente plusieurs parties de boîtier (7, 8) reliées l'une à l'autre en particulier par le biais d'une liaison vissée, en particulier une partie de boîtier proximale (7) et une partie de boîtier distale (8) et/ou en ce que dans une partie de boîtier (7), de préférence dans une partie de boîtier proximale (7), du corps de traitement (2), au moins un moyen d'éclairage, de préférence plusieurs moyens d'éclairage disposés de manière répartie uniformément sur la périphérie de la partie de boîtier proximale, en particulier des LED, sont prévus.

13. Boule d'amour (1) selon l'une quelconque des revendications précédentes, dans laquelle un boîtier (7, 8) du corps de traitement (2) se compose d'un matériau opaque à la lumière et/ou présente une surface opaque à la lumière et/ou en ce qu'au moins une ouverture de lumière (9) est prévue dans le boîtier (7) pour une émission de lumière vers l'extérieur et/ou en ce que plusieurs moyens d'éclairage et plusieurs ouvertures de lumière (9) sont prévus, dans laquelle, de préférence, une ouverture de lumière (9) séparée est associée à chaque moyen d'éclairage.

14. Boule d'amour (1) selon la revendication 13, dans laquelle l'ouverture de lumière (9) est fermée avec un élément de fermeture (10) en particulier de type disque en un matériau transparent à la lumière, dans laquelle, de préférence, l'élément de fermeture (10) présente une surface satinée et/ou en ce que l'élément de fermeture (10) se compose d'un matériau transparent, en particulier de plastique, de manière davantage préférée d'ABS, et/ou présente un tel matériau et/ou en ce que l'élément de fermeture (10) est réalisé sous forme de lentille convergente ou de lentille divergente.

15. Boule d'amour (1) selon l'une quelconque des revendications précédentes, dans laquelle au moins une partie de boîtier (7, 8) du corps de traitement (2), en particulier une partie de boîtier proximale (7) et une partie de boîtier distale (8), présente du côté extérieur une enveloppe (12, 13) et/ou un revêtement en un matériau plastique en particulier élastique, en particulier une enveloppe et/ou un revêtement de silicone, de caoutchouc, de latex et/ou de polyuréthane et/ou en ce qu'une enveloppe (13) et/ou un revêtement au niveau de l'extrémité distale (4) du corps de traitement (2) et une section de rappel (5) sont réalisés d'une seule pièce et/ou en ce que le corps de traitement (2) est enveloppé et/ou revêtu du côté extérieur sensiblement sur toute la surface.
